# EUROPEAN PATENT SPECIFICATION

(11) **EP 2 142 231 B1**
(45) Date of publication and mention of the grant of the patent: **17.09.2014**
(21) Application number: 08732242.6
(22) Date of filing: 14.03.2008
(51) Int. Cl.: A61L 31/04, A61L 31/10, A61L 31/16

(54) **A STENT COMPRISING SURFACE-BINDING CELL ADHESION POLYPEPTIDES AND A METHOD FOR COATING A STENT**
EIN STENT UMFASSEND OBERFLÄCHENBINDENDE ZELLADHÄSIONSPOLYPEPTIDE UND EIN VERFAHREN ZUR BESCHICHTUNG EINES STENTS
STENT COMPRENANT DES POLYPEPTIDES D'ADHÉSION CELLULAIRE SE LIANT À UNE SURFACE ET PROCÉDÉ POUR APPLIQUER UN REVÊTEMENT SUR UN STENT

(30) Priority: 15.03.2007 US 894944 P
(43) Date of publication of application: 13.01.2010
(73) Proprietor: Boston Scientific Limited, Christ Church (BB)
(72) Inventor: BENCO, John, S., Holliston, MA 01746 (US)
(74) Representative: Vossius & Partner
(86) International application number: PCT/US2008/057061
(87) International publication number: WO 2008/113005

(56) References cited:
- US-A1- 2003 229 393
- US-B1- 6 497 729
- GERMANIER Y ET AL: "enhanced bone apposition around biofunctionalized sandblasted and acid-etched titanium implant surfaces, a histomorphometric study i miniature pigs" CLINICAL ORAL IMPLANTS RESEARCH, MUNKSGAARD INTERNATIONAL PUBLISHERS, DK, vol. 17, 1 January 2006 (2006-01-01), pages 251-257, XP002419124 ISSN: 0905-7161
- JEFFREY LAWRENCE DALSIN: "Mussel Adhesive-Inspired Surface Modification for the Preparation of Nonfouling Biomaterials" December 2004 (2004-12), , EVANSTON, ILLINOIS, USA , XP002533788 pages 1-6 page 11 pages 34-41 pages 142-149 page 164, paragraph 1 - page 168, paragraph 1
- OPIE SHAUN R ET AL: "Local endovascular delivery, gene therapy, and cell transplantation for peripheral arterial disease." JOURNAL OF ENDOVASCULAR THERAPY : AN OFFICIAL JOURNAL OF THE INTERNATIONAL SOCIETY OF ENDOVASCULAR SPECIALISTS DEC 2004, vol. 11 Suppl 2, December 2004 (2004-12), pages II151-II162, XP002534318 ISSN: 1526-6028

## Description

### Background

Medical devices, such as implantable stents, have been coated with biologically active material. However, there are difficulties associated with effectiveness and/or stability of coatings on such medical devices under physiologically relevant conditions. These difficulties can be attributed to a number of factors. Bioactive coatings traditionally have relied on non-specific adsorption phenomena, such as hydrophobic interactions and/or polar interactions (e.g., hydrogen bonds) for adherence to surfaces of the medical device. Unfortunately, these mechanisms of adhesion are thermodynamically weak having estimated binding constants of ≈10⁹. See, Vadgama, Ed., Surfaces and Interfaces for Biomaterials, Woodhead Publishing LTD., Cambridge, England (2005) pp 770. As such, new methods are needed to improve the adhesion of bioactive molecules and/or coatings to surfaces of medical devices.

US 6 497 729 B1 discloses :an implantable medical device comprising: a substrate having a first surface; and a conjugate or complex disposed on at least a portion of the first surface, the complex/conjugate comprising a tissue response modifier polypeptide and a surface adhesive. US 6 497 729 B1 discloses additionally that the medical device is a stent.

GERMANIER Y ET AL: "enhanced bone apposition around biofunctionalized sandblasted and acid-etched titanium implant surfaces, a histomorphometric study in miniature pigs", CLINICAL ORAL IMPLANTS RESEARCH, MUNKSGAARD INTERNATIONAL PUBLISHERS, DK, vol. 17, 1 January 2006 (2006-01-01), pages 251-257, disclosesa method for preparing an implantable medical device comprising: dipping the implantable medical device into a solution including surface-binding cell adhesion polypeptides, where the surface-binding cell adhesion polypeptides comprise at least one cell adhesion polypeptide and at least one surface-binding moiety, where the at least one surface-binding moiety has binding affinity for one or more metallic materials; and binding the surface-binding cell adhesion polypeptides to at least a portion of the surface, where the portion includes one or more of the metallic materials.

### Summary

The present disclosure provides a stent having a metallic surface and a coating on the metallic surface, the coating comprising: surface-binding cell adhesion polypeptides disposed on at least a portion of the metallic surface, wherein the surface-binding cell adhesion polypeptides comprise: a surface-binding moiety comprising a catechol moiety bound to the metallic surface; and a cell adhesion polypeptide attached to the surface-binding moiety, wherein the cell adhesion polypeptide comprises an extracellular matrix protein or a binding domain fragment thereof.

The present disclosure also provides a method for coating a stent having a metallic surface comprising: contacting a surface of the stent with surface-binding cell adhesion polypeptides, wherein the surface-binding cell adhesion polypeptides comprise at least one cell adhesion polypeptide and at least one surface-binding moiety comprising a catechol moiety, wherein the cell adhesion polypeptide comprises an extracellular matrix protein or a binding domain fragment thereof; and binding the surface-binding cell adhesion polypeptides to at least a portion of the metallic surface.

### Brief Description of the Drawings

In the drawings, wherein like numerals designate like parts throughout the same.
FIG. 1 is a schematic representation depicting greatly enlarged views of an example stent for use with the surface-binding cell adhesion polypeptides of the disclosure.
FIG. 2 is a schematic representation of an example of a medical device including a layer of surface-binding cell adhesion polypeptides.
FIG. 3 is a schematic representation of another example of a surface-binding cell adhesion polypeptide.
FIG. 4 is a schematic representation of a further example of a layer of surface-binding cell adhesion polypeptides.
FIG. 5 is a graph showing Human coronary artery endothelial cell (HCAEC) migration on stainless steel strips alone compared to stainless steel strips coated with a peptide of the disclosure.
FIG. 6 is a graph showing the change in OD compared to the coating concentration of a polypeptide of the disclosure.
FIG. 7 is a graph showing the affinity of a polypeptide of the disclosure for stainless steel over time.
FIG. 8 is a graph showing the affinity of a polypeptide of the disclosure to stainless steel compared to pH.
FIG. 9 is a graph showing the impact of EtO sterilization on the conformation of a polypeptide of the disclosure when coated on a stainless steel substrate. The asterisk (*) indicates statistical significance.
FIG. 10 are images of Liberte WH stents coated with a polypeptide of the disclosure.
FIG. 11 is a graph showing the equilibrium binding constant of a polypeptide of the disclosure on stainless steel determined using the Langmuir Isotherm.

### Detailed Description

For the following defined terms, these definitions shall be applied, unless a different definition is given in the claims or elsewhere in this specification.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Standard single letter and three letter abbreviations are used herein to refer to naturally-occurring and non-naturally occurring amino acids either individually or connected within a polypeptide chain.

As used herein a "layer" of a given material is a region of that material whose thickness is small compared to both its length and width. As used herein a layer need not be planar, for example, taking on the contours of an underlying substrate. A layer can be discontinuous, providing only partial coverage of the underlying substrate. Terms such as "film," "layer" and "coating" may be used interchangeably herein.

The present disclosure provides a stent having a metallic surface and a coating on the metallic surface, the coating comprising:
surface-binding cell adhesion polypeptides disposed on at least a portion of the metallic surface, wherein the surface-binding cell adhesion polypeptides comprise:
   a surface-binding moiety comprising a catechol moiety bound to the metallic surface; and
   a cell adhesion polypeptide attached to the surface-binding moiety, wherein the cell adhesion polypeptide comprises an extracellular matrix protein or a binding domain fragment thereof.

The surface-binding cell adhesion polypeptides adhere or bind to a portion of the substrate surface better than cell adhesive peptides not attached to at least one surface-binding moiety. The present disclosure also provides a method for forming a layer of surface-binding cell adhesion polypeptides on at least a portion of a substrate surface.

A metallic stent having surface-binding cell adhesion polypeptides disposed on at least portion of a substrate surface thereof may promote adhesion and/or growth of epithelial cells onto the medical device or a portion thereof when such a device is implanted into a patient's vasculature or tissue. The recruitment of epithelial cells (e.g., endothelial cells) onto an implanted medical device may effectively "hide" the implanted device from the patient's body thereby reducing likelihood of negative effects, such as thrombosis or restenosis as well as immune response and rejection. For example, a layer of surface-binding cell adhesion polypeptides on a substrate surface, such as the lumen of a vascular implantable device (e.g., a stent) promotes attachment and/or proliferation of endothelial cells over the luminal surface of the stent when such a device has been implanted into a patient's blood vessel. By facilitating attachment and/or growth of endothelial cells on the luminal layer of surface-binding cell adhesion polypeptides, the stent is separated from the flowing blood, thereby reducing likelihood of turbulence and/or immune response, as well as reducing likelihood of thrombosis and/or restenosis.

Surface-binding cell adhesion polypeptides may preferentially bind to at least a portion of at least one surface of a substrate. Often surface-binding cell adhesion polypeptides bind to at least a portion of one or more surfaces of a substrate. Typically, a surface-binding cell adhesion polypeptide preferentially binds to an inner (e.g., luminal) surface of a substrate of an implantable medical device. In some instances, a surface-binding cell adhesion polypeptide preferentially binds to at least one inner (e.g., luminal) surface and at least one outer (e.g., abluminal) surface of a substrate of an implantable medical device.

The surface-binding catechol moiety of a surface-binding cell adhesion polypeptide is selected dependent on the choice of materials in the substrate surface or portion thereof to which binding is preferred, how much a surface-binding moiety assists binding of the surface-binding cell adhesion polypeptides to a material found in at least a portion of a surface of the substrate, and the environment in which surface-binding cell adhesion polypeptides are bound to the substrate surface.

Additionally, a surface-binding cell adhesion polypeptide includes a surface-binding moiety which preferentially binds to a non-metallic inorganic portion of a substrate surface.

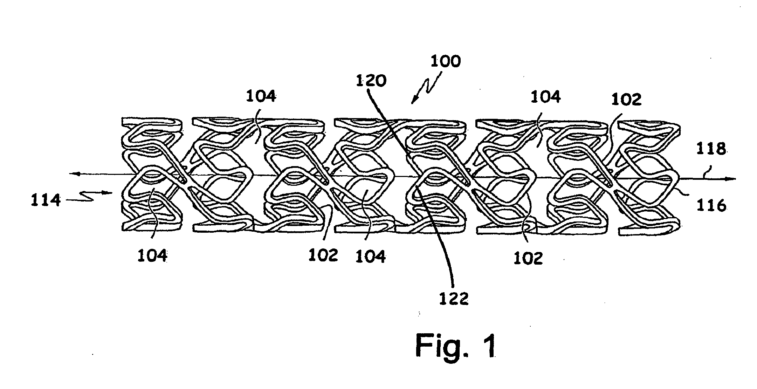

Further description of the surface-binding cell adhesion polypeptides is presented in relationship to an implantable medical device for purposes of illustration rather than limitation. Referring now to the drawings, FIG. 1 shows an implantable medical device, stent 100 which is at least partially constructed of a single member 102, or a plurality of interconnected struts or connectors (not shown). Member 102 defines a proximal opening 114, a distal opening 116 and a flow path 118 there between. Member 102 also defines cell openings 104, which are in fluid communication with the flow path 118. Member 102 is considered to have an inner or luminal surface 120 that faces flow path 118, as well as an outer or abluminal surface 122.

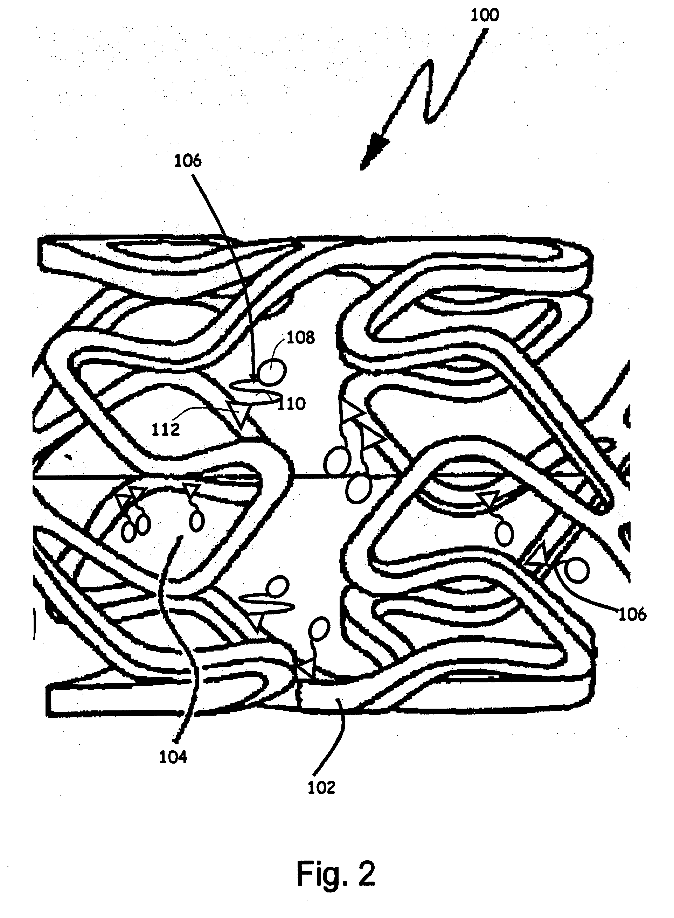

An example of surface-binding cell adhesion polypeptides bound to at least a portion of a surface of a substrate is shown in the greatly enlarged schematic representation of a section of stent 100 in FIG. 2. As described above, stent 100 is composed of at least one member 102, which serves as a substrate for surface-binding cell adhesion polypeptides 106 disposed on at least a portion of luminal surface 120. As shown in FIG. 2, a surface-binding cell adhesion polypeptide 106 may include a cell adhesive polypeptide 108 attached via a linker region 110 onto surface-binding moiety 112.

### Surface-binding moieties

A surface-binding cell adhesion polypeptide of the present disclosure includes a surface-binding catechol moiety which is selected at least in part for its ability to bind to one or more materials found in at least a portion of a surface of a substrate of an implantable medical device. The choice of surface-binding moiety depends upon the material(s) found in one or more surfaces of an implantable medical device, how much a particular surface-binding moiety assists adherence of a surface-binding cell adhesion polypeptide to one or more of those materials, and the environment where being bound to a portion of a substrate surface of an implantable medical device is desired.

Often a surface-binding moiety suitable for use to bind to a portion of a substrate surface of an implantable medical device has usable binding affinity to one or more materials found in a metallic surface. A metallic surface typically includes one or more materials, such as, stainless steel, titanium alloys, tantalum, cobalt-chrome alloys, or other Fe-, Cr-, or Ti-containing alloys. For example, a surface-binding moiety having usable binding affinity for Cr is expected to bind portions of a substrate surface of an implantable medical device which contain Cr, such as cobalt-chrome alloys, stainless steel, or other Cr-containing alloys.

Alternatively or additionally, a surface-binding moiety suitable for use to bind to a substrate surface of an implantable medical device has usable binding affinity to one or more materials found in a non-metallic inorganic surface. A non-metallic inorganic surface includes one or more materials, such as metal oxide ceramics, including aluminum oxides and transition metal oxides (e.g., oxides of titanium, zirconium, hafnium, tantalum, molybdenum, tungsten, rhenium, iron, niobium, and iridium); silicon; silicon-based ceramics, such as those containing silicon nitrides, silicon carbides and silicon oxides (sometimes referred to as glass ceramics); calcium phosphate ceramics (e.g., hydroxyapatite); carbon; and carbon-based, ceramic-like materials such as carbon nitrides.

Determination of an equilibrium binding constant, such as Kₐ, is one way for determining usable binding affinity or otherwise characterize the binding interaction between surface-binding cell adhesion polypeptides (or more specifically the surface-binding moieties therein) and the portion of the surface of a substrate that surface-binding cell adhesion polypeptides are disposed on. Often, the Kₐ between a surface-binding cell adhesion polypeptide (or more specifically the surface-binding moieties therein) and a substrate surface of an implantable medical device is equal to or greater than 10¹⁰. Typically, the Kₐ between a surface-binding cell adhesion polypeptide (or more specifically the surface-binding moieties therein) and a substrate surface of an implantable medical device is greater than 10¹². In some instances, the Kₐ between a surface-binding cell adhesion polypeptide (or more specifically the surface-binding moieties therein) and a substrate surface of an implantable medical device is greater than 10¹⁵. Methods that may be used for determination of Kₐ are known. See for example, Sever et al. (2006) Dalton Trans. 813-822, incorporated herein by reference.

Another way to characterize usable binding affinity is through assessment of binding of surface-binding cell adhesion polypeptides to a portion of a surface of a stent or other implantable medical device, in a sufficient quantity and for a sufficient time period, to provide therapeutic benefit. Therapeutic benefit refers to treatment of a diseases or conditions (i.e., the reduction or elimination of symptoms associated with a disease or condition, or the substantial or complete elimination of a disease or condition). To provide therapeutic benefit, typically the binding of surface-binding cell adhesion polypeptides and/or the adhesive moieties for metal surfaces is assessed at least under biologically relevant conditions. Biologically relevant conditions are defined for the purposes of the present disclosure as buffered aqueous solution, pH 7.0-7.6 between 25-40°C. In particular, biologically relevant conditions are those encountered in the bloodstream of a living mammal. Often, the biologically relevant conditions are those of the bloodstream of a living human patient. For example, the biologically relevant conditions may be those conditions found in the arterial blood of a living human heart.

Often, usable binding affinity refers to an expectation that at least 1% of the surface-binding cell adhesion polypeptides, disposed on a portion of a substrate surface of an implantable medical device, remain bound to a portion of a substrate surface of an implantable medical device after exposure to biologically relevant conditions for a time period of at least 5 minutes. Typically, usable binding affinity refers to an expectation that at least 1% of the surface-binding cell adhesion polypeptides, disposed on a portion of a substrate surface of an implantable medical device, remain bound to a portion of a substrate surface of an implantable medical device after exposure to biologically relevant conditions for a time period of at least 2 hours. In some instances, usable binding affinity refers to an expectation that at least 1% of the surface-binding cell adhesion polypeptides, disposed on a portion of a substrate surface of an implantable medical device, remain bound to a portion of a substrate surface of an implantable medical device after exposure to biologically relevant conditions for a time period of at least 24 hours. In an example, usable binding affinity refers to an expectation that at least 1% of the surface-binding cell adhesion polypeptides, disposed on a portion of a substrate surface of an implantable medical device, remain bound to a portion of the substrate surface after exposure to biologically relevant conditions for a time period after a time period of at least 90 days.

In other instances, usable binding affinity refers to an expectation that at least 50% of the surface-binding cell adhesion polypeptides, disposed on a portion of a substrate surface of an implantable medical device, remain bound to a portion of a substrate surface of an implantable medical device after exposure to biologically relevant conditions for a time period of at least 5 minutes. Sometimes, usable binding affinity refers to an expectation that at least 50% of the surface-binding cell adhesion polypeptides, disposed on a portion of a substrate surface of an implantable medical device, remain bound to a portion of a substrate surface of an implantable medical device after exposure to biologically relevant conditions for a time period of at least 2 hours. Occasionally, usable binding affinity refers to an expectation that at least 50% of the surface-binding cell adhesion polypeptides, disposed on a portion of a substrate surface of an implantable medical device, remain bound to a portion of a substrate surface of an implantable medical device after exposure to biologically relevant conditions for a time period of at least 24 hours. In an example, usable binding affinity refers to an expectation that at least 50% of the surface-binding cell adhesion polypeptides, disposed on a portion of a substrate surface of an implantable medical device, remain bound to a portion of a substrate surface of an implantable medical device after exposure to biologically relevant conditions for a time period of at least 90 days.

In some instances, it may be desirable to think about useable binding affinity in terms of EC₅₀, the effective concentration at which half of the binding sites on the substrate surface are occupied by adsorbate (surface-binding cell adhesion polypeptide). In some instances, a useable binding affinity can refer to an EC₅₀ that is substantially less (e.g., by at least 2 orders of magnitude, or at least 3 orders of magnitude) than the EC₅₀ for a non-specific bioactive coatings (which generally have an EC₅₀ in the range of 100 µM). Alternately, a useable binding affinity can refer to an EDC₅₀ that is less than 10µM, less than 1 µM, less than 0.5 µM, or less than 0.3 µM or between 0.2 µM and 0.5µM or between 0.25 µM and 0.35 µM. The useable binding affinity of a surface-binding cell adhesion polypeptide on a substrate surface may also refer to an expectation that the EC₅₀ remain substantially constant over a biologically relevant period of time. For example, a useable binding affinity may refer to an EC₅₀ that changes less than 25%, less than 20%, less than 15%, less than 10%, less than 5% or less than 1% over a relevant period of time, for example, within a 24 hour time period, a 10 hour time period, a 5 hour time period, or a 1 hour time period. In some instances, useable binding affinity may refer to an EC₅₀ that changes less than 15% over a period of 24 hours.

In other instances, it may be useful to think about useable binding affinity in terms of EC₅₀ over a pH range. For example, a useable binding affinity may refer to an EC₅₀ that remains substantially constant, for example, remains within 50%, 25%, 20%, 15%, 10%, 5% or 1% of the original EC₅₀ as the pH is shifted between a pH of 4 and a pH of 7. In some cases, a useable binding affinity may refer to an EC₅₀ that remains substantially constant, within 1 µM, 0.5 µM, 0.4 µM, 0.3 µM, 0.2 µM or 0.1 µM as the pH is shifted between a pH of 4 and a pH of 7. In some instances, the EC₅₀ at pH 7 may be less than 1.5 µM, less than 1.0 µM, between 0.5 µM and 1.5 µM, or between 0.5 µM and 1.0 µM.

Another important consideration when determining useable binding affinity of a coating for an implantable medical device is whether the binding affinity remains substantially constant when the coated device undergoes sterilization. Therefore, in some instances, useable binding affinity may refer to a binding affinity, for example, as determined by measuring the optical density (OD) of a chromogenic label (see Example 2), wherein the binding affinity decreases by less than 25%, 20%, 15%, 10%, 5% or 1%, or between 1% and 25%, between 5% and 20% or between 10% and 20% after sterilization.

In still other instances, useable binding affinity may refer to the surface coverage of the surface-binding cell adhesion polypeptide on a substrate surface. Surface coverage (*θ*) can be defined as the fraction of adsorption sites on a substrate surface that are occupied by the adsorbate (in this case, the surface-binding cell adhesion polypeptide). Surface coverage can be difficult to measure experimentally. However, surface coverage can be calculated and generally ranges from *θ*=Kc (where K=equilibrium binding constant; and c= concentration) to *θ*=1. Surface coverage can also be determined, for example, using an algorithm such as the Langmuir isotherm, a function that correlates the amount of adsorbate (in this case, the surface-binding cell adhesion polypeptide) on the adsorbent (or the substrate surface) with its concentration.
Langmuir isotherm: 1/m = 1/b +1/(bKc), where m is the adsorbed mass, b is a constant, K is the equilibrium binding constant and c is the peptide concentration.

In some instances, a useable binding affinity may refer to an adsorption pattern that fits a Langmuir isotherm, generally suggesting that the surface coverage is a monolayer.

Relative or absolute quantities of surface-binding cell adhesion polypeptides bound to a portion of a substrate surface or binding constants for binding of surface-binding cell adhesion polypeptides bound to a substrate surface may be measured by known assays, such as colorimetric type assays, wherein the surface-binding cell adhesion polypeptides are labeled for ready detection. The bound quantity of surface-binding cell adhesion polypeptides may be assessed before, after, and/or during exposure to a biologically relevant environment. For example: a biotin tag is attached to each surface-binding cell adhesion polypeptide. The biotin-surface-binding cell adhesion polypeptides are disposed on at least a portion of a substrate surface which is representative of a medical device or is a medical device as described below. An excess of labeled-streptavidin is added for binding to the biotin tag. The coated substrate surface is removed from the aqueous solution, rinsed to remove any unbound labeled-streptavidin. The substrate surface is subsequently read to determine an initial amount of surface-binding cell adhesion polypeptides bound on the substrate surface. Next, the substrate surface with bound surface-binding cell adhesion polypeptides is placed in a biologically relevant environment, described above, for example a buffered aqueous solution at pH 7.3, 37°C for 24 hours. The substrate is removed from the aqueous solution, rinsed to remove any unbound labeled-streptavidin and biotin-surface-binding cell adhesion polypeptides. The substrate surface is subsequently read to determine the amount of surface-binding cell adhesion polypeptides that remain bound to the substrate surface. The label, for example a fluorescent molecule, AU nanoparticle, or other moiety which is readily detected and quantitated, using known spectroscopic techniques. Alternatively, or additionally, Comparisons with cell adhesion polypeptide coatings lacking adhesive moieties are similarly performed. *See also,* Tsai et al. (2005) Chem. Commun. 4273-4275, incorporated herein by reference.

Experiments such as those described above can be used to determine concentration ranges for application of a surface-binding cell adhesion polypeptide to a substrate surface. In some instances, it may be desirable to provide the surface-binding cell adhesion polypeptide at a concentration of at least about 0.01 µM. In other instances, it may be desirable to provide a concentration of at least about 1 µM. More typically, a coating obtained by applying a surface-binding cell adhesion polypeptide to a substrate at a concentration of at least 10µM, or 0.1 µM and 1000µM, between 1µM and 100µM, between 1 µM and 10µM, or between 10µM and 100 µM.

Yet another way to characterize usable binding affinity is through assessment of adhesion and/or growth of epithelial cells (e.g., endothelial cells) on a portion of a surface of a stent or other implantable medical device having surface-binding cell adhesion polypeptides disposed thereon. The ability of a portion of a surface to adhere and/or grow epithelial cells is expected to demonstrate usable binding affinity of the cell adhesion polypeptides disposed on said surface. Cellular adhesion to a surface may be measured, for example, by AFM (atomic force microscopy) techniques described in Razatos et al. (1998) PNAS 95:11059-11064, incorporated herein by reference. Growth of epithelial cells can also be determined by examining cell migration, for example, migratory activity of human coronary artery endothelial cells (HCAEC), as described in Example 1, below. In some instances, migratory activity of epithelial cells on a substrate surface coated with surface-binding cell adhesion polypeptides can be more than 1.2, 1.5, 1.7, 2, 3, or 5 times greater than on an uncoated substrate. In some instances, migratory activity of epithelial cells can be between 1.2 and 3 times, or between 1.5 and 2 times greater than on an uncoated substrate.

Adhesive moieties are readily attached to cell adhesion polypeptide by known peptide chemistries to form surface-binding cell adhesion polypeptides. Examples of adhesive moieties of surface-binding cell adhesion polypeptides of the present disclosure include: catechol moieties, polybisphosphonate moieties, and adhesive polypeptide segments.

### Catechol moieties

Surface-binding moieties of the present disclosure include, catechol moieties. Catechol moieties are attached to cell adhesion polypeptides. One or more catechol moieties may be attached to one or more cell adhesion polypeptides. Attachment may be directly to a cell adhesion polypeptide or indirect, for example via a linker. For example, a surface-binding moiety may includes a catechol moiety attached to one or more additional catechol moieties or other surface-binding moiety, and one or more cell adhesion polypeptides via one or more linkers.

Surface-binding moieties including one or more catechol moieties are expected to bind to metallic surfaces with useable affinity. Typically a catechol moiety binds with first-row transition metals with binding constant on the order of 10¹⁶ or higher. See, Sever et al. (2006) Dalton Trans. 813-822, incorporated herein by reference. Since the thermodynamic binding of catechol moieties is several orders of magnitude greater than is attributed to non-specific adsorption phenomena, such as hydrogen bonding, surface-binding cell adhesion polypeptides including catechol moieties are expected to exhibit greater stability disposed on a portion of a substrate surface under physiological conditions.

Often, the catechol moiety is as presented in formula I, 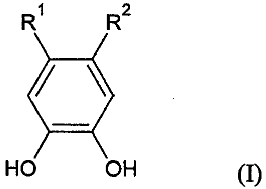 wherein R¹ and R² are individually selected from: a cell adhesive polypeptide, a linker, an additional surface-binding moiety, H, or combinations thereof, wherein at least one of R¹ and R² is directly or indirectly attached to at least one cell adhesion polypeptide. In certain instances, R¹ is a cell adhesive polypeptide or a linker, wherein the linker is attached to at least one cell adhesive polypeptide, and R² is H. For example, R¹ is a linker, wherein the linker is attached to at least one cell adhesive polypeptide, and R² is H.

For example, a catechol moiety useful in a surface-binding moiety is 3,4 dihydroxyphenylalanine, also referred to as DOPA. DOPA exhibits binding affinity with first-row transition metals with binding constant on the order of 10¹⁶ and is expected to provide useable binding affinity for surface-binding moieties and surface-binding cell adhesion polypeptides described herein. See, Sever et al. supra. DOPA moieties have also exhibited long term stability of several months on a metal surface under biologically relevant conditions. See, Statz et al. (2005) JACS 127:7972-7973, incorporated herein by reference. DOPA and derivatives thereof are represented below as formula II. 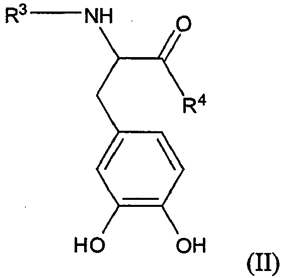 wherein R³ and R⁴ are individually selected from a cell adhesive polypeptide, a linker, an additional surface-binding moiety, H, or combinations thereof, wherein at least one of R³ and R⁴ is directly or indirectly attached to at least one cell adhesive polypeptide. In certain instances, R³ is a cell adhesive polypeptide or a linker, wherein the linker is attached to at least one cell adhesive polypeptide, and R⁴ is H. For example, R³ is a linker, wherein the linker is attached to at least one cell adhesive polypeptide, and R⁴ is H. Alternatively, R⁴ is a cell adhesive polypeptide or a linker, wherein the linker is attached to at least one cell adhesive polypeptide, and R³ is OH. For example, R⁴ is a linker, wherein the linker is attached to at least one cell adhesive polypeptide, and R³ is OH.

In certain instances, a surface-binding moiety includes the catechol moiety presented in formula III. The catechol moiety of formula III is also expected to provide useable binding affinity for surface-binding moieties and surface-binding cell adhesion polypeptides described herein. For example, a catechol moiety structurally similar to that presented in formula III has demonstrated stable binding to an TiO2 surface for a period of at least two days under conditions similar to those of human plasma. See, Zurcher et al. (2005) JACS 128:1064-1065, incorporated herein by reference.

In formula III, shown below: 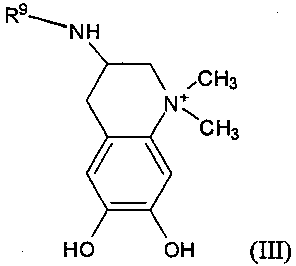 wherein R⁹ is a cell adhesive polypeptide, a linker, an additional surface-binding moiety, H, or combinations thereof, and wherein R⁹ is directly or indirectly attached to at least one cell adhesive polypeptide. For example, R⁹ is a linker, wherein the linker is attached to at least one cell adhesive polypeptide.

Catechol moieties are readily attached onto polypeptides, including cell adhesion polypeptides, peptide linkers, and/or additional catechol moieties, via standard peptide synthesis techniques. See for example, Hu et al. (2000) Tetrahedron Letters 41:5795-5798, incorporated herein by reference. One example of a catechol moiety of formula II attached to additional amino acids, which may be part of or attached to cell adhesion polypeptides, peptide linkers, and/or additional catechol moieties, is shown below as formula IV, 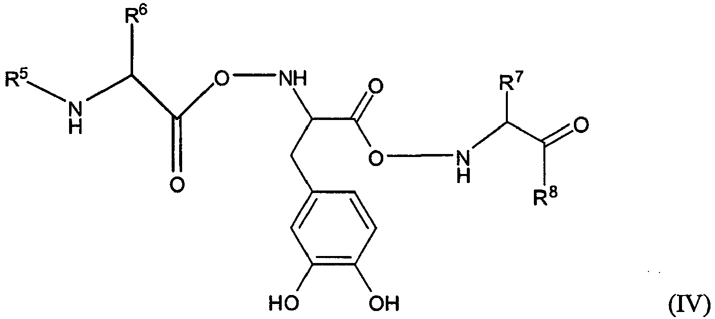 wherein R⁶ and R⁷ are naturally-occurring or non-naturally occurring amino acid side chains; and R⁵ is H, a linker, a catechol moiety, a cell adhesion polypeptide or combinations thereof; and R⁸ is OH, a linker, a catechol moiety, a cell adhesion polypeptide or combinations thereof; and wherein at least one of R⁵ and R⁸ is attached to at least one cell adhesive polypeptide. Further examples include wherein at least one of R⁵ and R⁸ is a linker, wherein the one or more linkers are attached to at least one cell adhesive polypeptide.

In certain instances, a surface-binding moiety includes at least two DOPA moieties of formula II joined by one or more amino acids. A specific example is presented in formula V, below, wherein the catechol surface-binding moiety is DOPA-Lys-DOPA-Lys- or (DOPA-Lys)₂. Formula V also presents the (DOPA-Lys)₂ surface-binding moiety attached to an example cell adhesion polypeptide, YIGSR. In Formula V, the cell adhesion polypeptide is attached to a peptide linker, (Ala-Aib)₃.

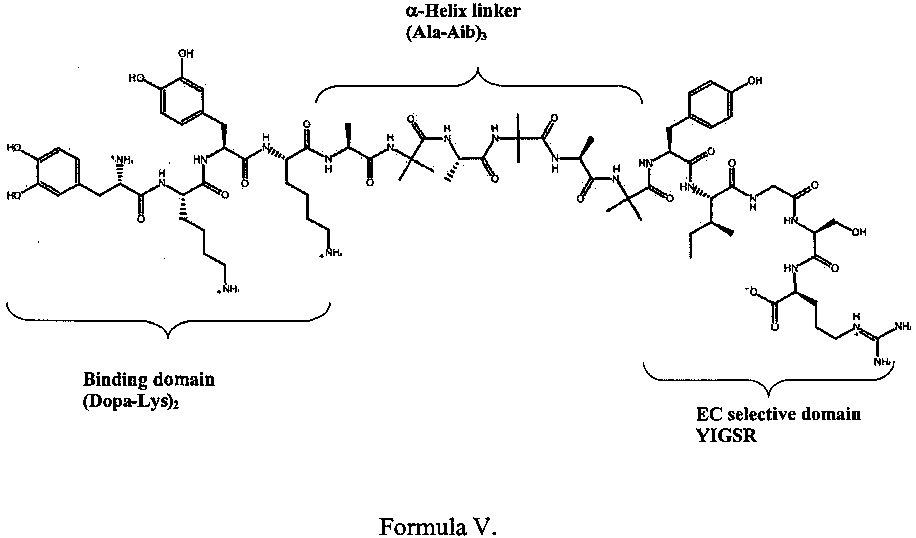

In general, adhesive moieties, including but not limited to catechol moieties, may be joined to each other and to cell adhesive polypeptides by linkers. Linkers may provide spacing from the metal surface, flexibility, secondary or higher level structure (e.g., *α*-helix) and reactive sites for attachment to a component or portion of the surface-binding cell adhesion polypeptide. Linkers between catechol adhesive moieties and cell adhesive polypeptides are preferably of sufficient length to allow spacing of the cell adhesion moiety from the substrate surface for binding to epithelial cells, such as endothelial cells. The spacing from the metal surface via linkers is also of sufficient length to avoid blocking (e.g., biofouling) of the cell adhesion moieties, for example by non-specific association of blood proteins or other components to the surface of the medical device. Linkers between catechol adhesive moieties and cell adhesive polypeptides are often at least 20Å in length. Typically, the linker between a catechol adhesive moiety and an attached cell adhesive polypeptide is within the range 20Å to 70Å, although alternatives are possible.

Example linkers suitable for use as described herein may be selected from any alkane, alkene, or aromatic molecules, any of which may be hetero-substituted with N, S, or O and combinations thereof, which are capable of attachment. Often, linkers are selected from polyethylene glycol, polyethyleneoxide, and polypeptides of naturally-occurring and non-naturally occurring amino acids. Typically, the linkers between catechol adhesive moieties and cell adhesive polypeptides are preferably polypeptides formed from at least 2 naturally-occurring and/or non-naturally occurring amino acids. In some instances, the linkers between catechol adhesive moieties and cell adhesive polypeptides are preferably polypeptides formed from 2 to 6 amino acids, although alternatives are possible. For example, linkers between adhesive moieties, such as individual catechol moieties may be shorter, for example, at least 1 naturally-occurring and/or non-naturally occurring amino acids.

### Cell adhesion polypeptides

As used herein, the term "cell adhesion polypeptides" refers to compounds having at least two amino acids per molecule which are capable of binding to epithelial cells (e.g., endothelial cells) via cell surface molecules, such as integrins, displayed on the surface of epithelial cells.

Typically cell adhesion polypeptides are any of the proteins of the extracellular matrix which are known to play a role in cell adhesion, including fibronectin, vitronectin, laminin, elastin, fibrinogen, and collagens, such as types I, II, and V. Additionally, the cell adhesion polypeptides may be any peptide derived from any of the aforementioned proteins, including derivatives or fragments containing the binding domains of the above-described molecules. Example peptides include those having integrin-binding motifs, such as the RGD (arginine-glycine-aspartate) motif, the YIGSR (tyrosine-isoleucine-glycine-serine-arginine) motif, and related peptides that are functional equivalents. For example, polypeptides containing RGD sequences (e.g., GRGDS) and WQPPRARI sequences are known to direct spreading and migrational properties of endothelial cells. See V. Gauvreau et al., Bioconjug Chem., 2005 Sep-Oct, 16(5), 1088-97. REDV tetrapeptide has been shown to support endothelial cell adhesion but not that of smooth muscle cells, fibroblasts, or platelets, and YIGSR pentapeptide has been shown to promote epithelial cell attachment, but not platelet adhesion. More information on REDV and YIGSR peptides can be found in U.S. Patent No. 6,156,572 and U.S. Patent Application No. 2003/0087111. See also, Boateng et al., RGD and YIGSR Synthetic Peptides Facilitate Cellular Adhesion Identical to That of Laminin and Fibronectin But Alter the Physiology of Neonatal Cardiac Myocytes, Am. J. Physiol. - Cell Physiol. 288:30-38 (2005), which is incorporated by reference herein. A further example of a cell-adhesive sequence is NGR tripeptide, which binds to CD13 of endothelial cells. See, e.g., L. Holle et al., "In vitro targeted killing of human endothelial cells by co-incubation of human serum and NGR peptide conjugated human albumin protein bearing alpha (1-3) galactose epitopes," Oncol. Rep. 2004 Mar; 11(3):613-6. The cell adhesion polypeptides may also be any of the peptides described in U.S. Patent Publication No. 20060067909 (West et al.), which is incorporated by reference herein. Alternatively the cellular adhesion polypeptides can be obtained by screening peptide libraries for adhesion and selectivity to specific cell types (e.g. endothelial cells) or developed empirically via Phage display technologies.

**Stents coated with surface-binding cell adhesive polypeptides**

Stents are provided which include a substrate having first and second surfaces, wherein at least a portion of the first substrate surface may bind to surface-binding cell adhesion polypeptides. Typically, stents include a substrate having first and second surfaces, and a layer of surface-binding cell adhesion polypeptides disposed on at least a portion of the first substrate surface. The surface-binding cell adhesion polypeptides may also bind to epithelial cells (e.g., endothelial cells) before, after, or during binding to the first substrate surface.

Examples of stents include, including coronary vascular stents, peripheral vascular stents, cerebral, urethral, ureteral, biliary, tracheal, gastrointestinal and esophageal stents, stent coverings, stent grafts.

As mentioned above, the implantable medical device is a stent which includes a substrate having a first surface, and a layer of surface-binding cell adhesion polypeptides disposed on at least a portion of the first substrate surface. Often, the implantable medical device has a first and a second surface, wherein a layer of surface-binding cell adhesion polypeptides is disposed on at least a portion of the first substrate surface to promote attachment and/or proliferation of epithelial cells (e.g., endothelial cells). A layer of surface-binding cell adhesion polypeptides may also be disposed on a least a portion of the second substrate surface and/or a drug-eluting coating may be disposed on at least a portion of the second substrate surface.

The implantable medical device is a stent, wherein the first and second surfaces are luminal and abluminal surfaces, and a layer of surface-binding cell adhesion polypeptides is disposed on at least a portion of the first substrate surface. Typically, the stent is an intravascular stent comprising an open lattice sidewall structure and designed for permanent implantation into a blood vessel of a patient. Examples include, an expandable stent, such as a self-expandable stent or balloon-expandable stent, having a tubular metal body having open ends and a sidewall structure having openings therein and a layer of surface-binding cell adhesion polypeptides disposed on at least a portion of the surface of the sidewall structure.

Often, surface-binding cell adhesion polypeptides, described above, preferentially bind to metallic portions of a surface of the stent via a surface-binding adhesive moieties attached onto the cell adhesion polypeptides. Since the layer of surface-binding cell adhesion polypeptides adheres to the metallic portions, it inherently conforms to the structure in a manner that preserves the openings, such as when the stent is expanding.

According to the present disclosure, surface-binding cell adhesion polypeptides are bound to at least a portion of the surface of stent for which the surface-binding cell adhesion polypeptides have useable binding affinity. Typically, surface-binding cell adhesion polypeptides are bound to a portion of a luminal surface of a stent.

Often, surface-binding cell adhesion polypeptides are bound to a portion or portions of an inner surface (e.g., luminal surface) and a portion or portions of an outer surface (e.g., abluminal surface) of a stent. In some instances, surface-binding cell adhesion polypeptides are bound to multiple portions and/or multiple surfaces of a stent. In those instances, surface-binding cell adhesion polypeptides.

The stents of the present disclosure include at least one substrate surface of metallic material for which surface-binding cell adhesion polypeptides of the present disclosure are selected to have useable binding affinity. The remainder of the implantable medical device may vary widely in composition and is not limited to any particular material.

Substrate materials for the stents disclosed herein can be selected from a range of biostable materials and biodisintegrable materials (i.e., materials that, upon placement in the body, are dissolved, degraded, resorbed, and/or otherwise removed from the placement site), including metallic materials (i.e., materials containing metals, typically 50 wt% or more, for example, from 50 wt% to 75 wt% to 90 wt% to 95 wt% to 97.5 wt% to 99 wt% or more) polymer/metallic-inorganic hybrids.

Specific examples of metallic materials may be selected, for example, from metals such as gold, iron, niobium, platinum, palladium, iridium, osmium, rhodium, titanium, tantalum, tungsten, ruthenium, and magnesium, among others, and metal alloys such as those comprising iron and chromium (e.g., stainless steels, including platinum-enriched radio-opaque stainless steel), niobium alloys, titanium alloys, alloys comprising nickel and titanium (e.g., Nitinol), alloys comprising cobalt and chromium, including alloys that comprise cobalt, chromium and iron (e.g., elgiloy alloys), alloys comprising nickel, cobalt and chromium (e.g., MP 35N), alloys comprising cobalt, chromium, tungsten and nickel (e.g., L605), alloys comprising nickel and chromium (e.g., inconel alloys), and biodisintegrable alloys including alloys of magnesium and/or iron (and their alloys with combinations of Ce, Ca, Zn, Zr and Li), among others.

Typically, stents suitable for coating with the surface-binding cell adhesion polypeptides have one or more metallic surfaces. Often, the metallic surfaces are selected from the group consisting of stainless steel, titanium alloys, tantalum, and cobalt-chrome alloys. In some instances, the metal surfaces of the stent or implantable medical device are at least partially oxidized prior to coating with surface-binding cell adhesion polypeptides. For example, certain alloys, such as stainless steel containing chromium oxidize under standard environmental conditions, and consequently may be considered pre-oxidized. In certain instances, the surface of the stent is treated to alter the chemical environment of the metal surface, for example by removing oxides.

The metal surface of the stent may be untextured or textured. Sometimes, the metal surface of the implantable medical device is porous, such as the porous stent surface described in U.S. Patent Publication No. 2005/0266040 (Gerberding), which is incorporated by reference herein. In certain instances, the surface of the implantable medical device is modified to impart texture, for example by providing a surface with topographical features. For example, the texture of the surface may be optimized (e.g., where the textural features are properly sized and spaced) such that endothelial cells are provided with a surface whereby proliferation to coverage (e.g., confluence) can occur rapidly. For instance, literature has shown that endothelial cells cultured on textured surfaces spread faster and appear more like cells in native arteries. See R.G. Flemming et al., Biomaterials 20 (1999) 573-588 and N. Fujisawa et al., Biomaterials 20 (1999) 955-962, both incorporated herein by reference. In addition, surfaces with topographical features have increased surface area. Typically, where a stent has a surface that is textured, the texture is imparted prior to coating the surface with the surface-binding cell adhesion polypeptides.

Examples of bifurcated stents and systems for delivery into vasculature include, but are not limited to, those shown and described in U.S. Patent Application No. 10/375,689, filed February 27,2003 and U.S. Patent Application No. 10/657,472, filed September 8,2003, both of which are entitled Rotating Balloon Expandable Sheath Bifurcation Delivery; U.S. Patent Application No. 10/747,546, filed December 29,2003 and entitled Rotating Balloon Expandable Sheath Bifurcation Delivery System; and U.S. Patent Application No. 10/757,646, filed January 13,2004 and entitled Bifurcated Stent Delivery System, the entire content of each being incorporated herein by reference. It should also be further noted that while stent 100 may be a standard "single vessel" stent such as is described above, or stent 100 may also be a bifurcated stent having a trunk or stem portion, with one or more leg portions and/or branch openings adjacent thereto. Such bifurcated stents and stent assemblies are well known in the art.

In addition, a stent having surface-binding cell adhesion polypeptides disposed on at least a portion of one substrate surface, may be configured to deliver one or more therapeutic agents to a delivery site, such as within the vessel or one or more areas adjacent thereto. For example, in FIG. 1, stent 100 includes a layer of surface-binding cell adhesion polypeptides disposed on at least a portion of luminal surface 120 and a layer of a drug-eluting coating may be disposed on at least a portion of abluminal surface 122. The drug-eluting coating includes at least one therapeutic agent and at least one polymer.

"Therapeutic agents," "biologically active agents," "drugs," "pharmaceutically active agents," "pharmaceutically active materials," and other related terms may be used interchangeably herein and include genetic therapeutic agents, non-genetic therapeutic agents and cells. A wide variety of therapeutic agents can be employed in conjunction with the implantable medical devices disclosed herein including those used for the treatment of a wide variety of diseases and conditions (i.e., the reduction or elimination of symptoms associated with a disease or condition, or the substantial or complete elimination of a disease or condition). Numerous therapeutic agents are listed below.

Suitable non-genetic therapeutic agents for use in connection with the implantable medical devices disclosed herein may be selected, for example, from one or more of the following: (a) anti-thrombotic agents such as heparin, heparin derivatives, urokinase, clopidogrel, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D- Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promoters; (g) vascular cell growth inhibitors such as growth factor inhibitors, growth factor receptor antagonists, transcriptional repressors, translation repressors, replication inhibitors, inhibitory antibodies, antibodies directed against growth factors, bifunctional molecules consisting of a growth factor and a cytotoxin, bifunctional molecules consisting of an antibody and a cytotoxin; (H) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, antimicrobial peptides such as magainins, aminoglycosides and nitrofurantoin; (m) cytotoxic agents, cytostatic agents and cell proliferation affectors; (n) vasodilating agents; (o) agents that interfere with endogenous vasoactive mechanisms, (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) beta-blockers, (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AN, AV, etc.).

Preferred non-genetic therapeutic agents include paclitaxel (including particulate forms thereof, for instance, protein-bound paclitaxel particles such as albumin-bound paclitaxel nanoparticles, e.g., ABRAXANE), sirolimus, everolimus, tacrolimus, Epo D, dexamethasone, estradiol, halofirginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well as derivatives of the forgoing, among others.

Exemplary genetic therapeutic agents for use in connection with the implantable medical devices disclosed herein include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor *α* and *β*, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or microparticles, with and without targeting sequences such as the protein transduction domain (PTD).

Cells for use in connection with the implantable medical devices disclosed herein include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular and other treatment regimens, for example, as agents targeting restenosis. Such agents are useful for the practice of the implantable medical devices disclosed herein and suitable examples may be selected from one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, *β*-antagonist such as propranolol and *α*/*β*-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and *β*-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (l) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-*β* pathway agents such as polyanionic agents (heparin, fucoidin), decorin, and TGF-*β* antibodies, EGF pathway agents such as EGF antibodies, receptor antagonists and chimeric fusion proteins, TNF-*α* pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional therapeutic agents useful for the practice of the implantable medical devices disclosed herein are also disclosed in U.S. Patent No. 5,733,925 to Kunz et al.

The therapeutic-agent-eluting polymeric layer will typically comprise, for example, from 1 wt% or less to 2 wt% to 5 wt% to 10 wt% to 25 wt% to 50 wt% or more of a single therapeutic agent or of a mixture of therapeutic agents within the layer. Therapeutic agents may be selected, for example, from those listed below, among others.

The drug-eluting coating will also typically comprise, for example, from 50 wt% or less to 75 wt% to 90 wt% to 95 wt% to 97.5 wt% to 99 wt% or more of a single polymer or a mixture polymers within the layer. Polymers may biodegradable or biostable and may be selected, for example, from those described above for use in substrates and from those described below in conjunction with textured polymeric layers, among others.

The thickness of the drug-eluting coating may vary widely, typically ranging from 10 nm to 25 nm to 50 nm to 100 nm to 250 nm to 500nm to 1 *µ*m to 2.5 *µ*m to 5 *µ*m to 10 *µ*m to 20 *µ*m or more in thickness.

Drug-eluting coating may be disposed on substrates using any suitable method known in the art. For example, where the layer contains one or more polymers having thermoplastic characteristics, the layer may be formed, for instance, by (a) providing a melt that contains polymer(s), therapeutic agent(s), and any other optional species desired and (b) subsequently cooling the melt. As another example, a layer may be formed, for instance, by (a) providing a solution or dispersion that contains one or more solvent species, polymer(s), therapeutic agent(s), and any other optional species desired and (b) subsequently removing the solvent species. The melt, solution or dispersion may be disposed on at least a portion of a substrate surface, for example, by extrusion onto the substrate, by coextrusion along with the substrate, by roll-coating the substrate, by application to the substrate using a suitable application device such as a brush, roller, stamp or ink jet printer, by dipping the substrate, spray coating the substrate using spray techniques such as ultrasonic spray coating and electrohydrodynamic coating, among other methods. In certain instances, another surface of the substrate is masked to prevent the therapeutic-agent-eluting polymeric layer from being applied thereon.

### Application methods

Surface-binding cell adhesion polypeptides are bound to at least a portion of the surface of a stent. In certain instances, surface-binding cell adhesion polypeptides are bound to all surfaces of the surface of a stent. Occasionally, surface-binding cell adhesion polypeptides are bound to the inner surface and outer surface of the surface of a stent. Often, surface-binding cell adhesion polypeptides are bound to the inner surface of a stent.

In an example, an inner surface of a stent is a luminal surface and the outer surface of an implantable medical device is an abluminal surface.

Surface-binding cell adhesion polypeptides are bound to at least a portion of the surface of a stent and also may also be bound to an epithelial (e.g., endothelial) cell without limitation as to order in which the binding interactions take place. The surface-binding cell adhesion polypeptides may bind to epithelial cells (e.g., endothelial cells) before, after, or during binding to a portion of a substrate surface. Typically, surface-binding cell adhesion polypeptides are disposed on at least a portion of the surface of a stent prior to binding to epithelial cells. Often, surface-binding cell adhesion polypeptides are disposed on at least a portion of the surface of a stent prior to use of the device in a patient, i.e., prior to implantation. Alternatively, surface-binding cell adhesion polypeptides may be bound to epithelial cells prior to binding of the surface-binding cell adhesion polypeptides with at least a portion of the surface of a stent. In such instances, the implantation of the device in a patient may be before or after binding the surface-binding cell adhesion polypeptides with the surface of a stent.

The cell adhesion polypeptides can be applied onto the surface of the stent in various ways, including the use of coating methods that are known in the art. In general, the surface-binding cell adhesion polypeptides are provided in a mixture with solvent which is brought into contact with the stent. The coating may be applied with the stent fully expanded. Also, the coating may be applied as the stent is rotated. See for example, US 2005/01584050. Contact time with the mixture is selected to allow the surface-binding cell adhesion polypeptides to form a coating by binding to the stent. Then the solvent is removed, typically by evaporation. The stent may be contacted with surface-binding cell adhesion polypeptides one or more times for coating formation.

The concentration of surface-binding cell adhesion polypeptide in the mixtures is typically from about 0.1 to about 100 µM. Suitable solvents include aqueous solutions, which may include additives to speed evaporation. The solvent is typically a buffered solution between pH 4.0 to 9.0 suitable for solvating the surface-binding cell adhesion polypeptides described herein at a concentration of at least 1 µM.

Surface-binding cell adhesion polypeptides may be applied by dipping the stent in a mixture of surface-binding cell adhesive polypeptides in a solvent. This method may be used to coat all surfaces of the stent. Alternatively, the coating can be applied to surfaces the stent by spraying a coating composition comprising a mixture of surface-binding cell adhesive polypeptides in a solvent. All surfaces or selected areas of the stent may be spray coated.

For example, the surface-binding cell adhesive polypeptides may be sprayed onto the stent by a conventional electrostatic spraying process, resulting in charged peptide-containing droplets being deposited onto the medical device. As the coating fluid dries, the polypeptides remain adhered to the medical device by inter-molecular bonding with the side-chain groups on the polypeptides. In certain instances, the deposited polypeptides may form a monolayer on the surface of the medical device, such as a Langmuir monolayer or a self-assembling monolayer as described in Van Alsten, Self-Assembled Monolayers on Engineering Metals: Structure, Derivation, and Utility, Langmuir 15:7605-14 (1999).

### WORKING EXAMPLES

### Example 1: HCAEC migration

Migration and proliferation of endothelial cells on biomaterials can impact the rates of reendothelization and angiogenesis, which can be important during the development and success of implantable medical devices. In this experiment, the effect of a surface-binding cell adhesion polypeptide on the motility of human coronary artery endothelial cells (HCAEC) on a stainless steel substrate was examined *in vitro.* The surface-binding cell adhesion polypeptide used in this example included DOPA as a surface binding moiety, a (ALA-Aib)3 linker, and the recognition sequence for laminin receptors (YIGSR) as the cell adhesion polypeptide [(DOPA)2-(ALA-Aib)3-YIGSR-OH, hereinafter PEPTIDE-1] (synthesized at the MGH Peptide/Protein Core Facility, Harvard Medical School, Boston, MA).

Human coronary artery endothelial cells (HCAEC, available from Cell Applications, Inc., San Diego, CA) were used in this study. The substrates were sterile stainless steel strips [3.5" x 0.308" x 0.031"] or sterile stainless steel strips coated with PEPTIDE-1.

Collagen gels were prepared using the following protocol: One 10 mg vial of high concentration (HC) Type I collagen (Rat tail, from BD Biosciences, San Jose, CA) was diluted with distilled sterile H₂O to a final volume of 38 ml and mixed thoroughly without introducing bubbles (by pipetting up and down). The collagen solution was then transferred to 6 square Petri dishes (6 ml per Petri dish). To minimize volume loss of the viscous collagen solution, the same pipet was used to transfer all of the solution. The bottom of the Petri dish was coated with the collagen solution by gentle tilting and light shaking. Large bubbles were popped and removed. Small bubbles were pushed to the side of the dish using a pipet tip.

An ammonium hydroxide chamber was prepared by saturating a folded paper towel with ammonium hydroxide and placing it inside a Tupperware container that was sufficiently large to hold at least one Petri dish. The uncovered Petri dish was placed on a level surface in the ammonium hydroxide chamber and the lid closed tightly. The Petri dish was exposed to the ammonia vapor in the chamber for 5 minutes and then removed. 15 ml of distilled sterile H2O (DS H2O) was added to the top of the collagen gel and allowed to sit at room temperature for 15 minutes. The gel was then gently washed 2X with an additional 15 ml of DS H2O and then allowed to sit in a hood overnight in the DS H2O. Alternately, the Petri dishes were wrapped and stored at 4°C for up to two weeks.

On the morning of the experiment, the gels were gently washed 3X with 10 ml HCAEC growth medium (Cell Applications Inc., San Diego, CA), allowing 5 minutes at room temperature (RT) between washes. One T-75 cell culture flask (BD Falcon) containing confluent HCAEC grown in HCAEC growth medium were harvested by trypsinization per Petri dish to be used in the assay. All cells to be plated on a Petri dish were combined in a master cell suspension in HCAEC growth medium. The master cell suspension contained at least 10 ml of suspension for each Petri dish. 10 ml of cell suspension was then plated on each Petri dish and incubated at 37°C for approximately 4 to 5 hours.

After a monolayer of cells was established, the plates were checked for proper distribution and spreading. The plates were then aspirated and 15 ml HCAEC growth medium was added back to the plates.

Sterile stainless steel strips were coated with PEPTIDE-1 using the following protocol: 1.5 ml coating solution containing Dulbecco's Phosphate Buffered Saline with Calcium and Magnesium (Invitrogen, Carlsbad, CA) and 10 µM PEPTIDE-1 was prepared for each stainless steel strip to be coated. An X was etched near the top on one side of each strip using a pair of tweezers to denote the back side of the strip. The stainless steel strips were placed in a polystyrene tube and at least 1.5 ml of the coating solution was added to each tube. The tube was then placed on its side in shaker with the "X" side of the stainless steel strip facing upwards. The tubes were shaken at approximately 100 RPM at 4°C overnight.

3 (triplicate) stainless steel strips were pressed into the gels of each Petri dish. Since the strips did not fit into the Petri dish when positioned straight up and down, the strips were pressed into the gel at a slight angle to the dish. If the strip did not wet completely, the dish was rocked until medium covered the strip. The strips and dishes were returned to the 37°C incubator for 2 days (48 hours).

HCAEC migratory activity was quantified using the following protocol:
Calcein-AM stain was prepared by obtaining one 50 *µ*g vial of stain for each 12.5 ml of staining solution (Hanks's Balanced Salt Solution - HBSS) desired (approximately 9-12.5 ml of staining solution typically can stain 6 stainless steel strips in a square Petri dish). 20 *µ*l of Dimethyl Sulfoxide (DMSO) and 150 *µ*l of warm HBSS staining solution was added to each 50 *µ*g vial of calcien-AM and mixed slowly. This solution was then transferred into a square Petri dish (100x15-mm, Falcon), along with 12.5 ml of HBSS. The vial was washed using HBSS. HBSS was mixed with stain thoroughly, and approximately 7-10 ml of the stain was transferred to each square Petri dish, which was then covered with foil and put aside.

The stainless steel strips were removed from the gels, washed 2X with HBSS, and then submerged in the staining solution. After the strips were incubated at 37°C for 60 minutes, they were washed in HBSS 3X, tapped dry and read with a Spectramax M5 with the following settings: top read fluorescence; EX/EM = 485/530nm with auto cutoff; and well scan 9 positions. Fluorescence readouts obtained for stainless steel strips coated with peptide were normalized by readouts obtained from uncoated (control) stainless steel strips.

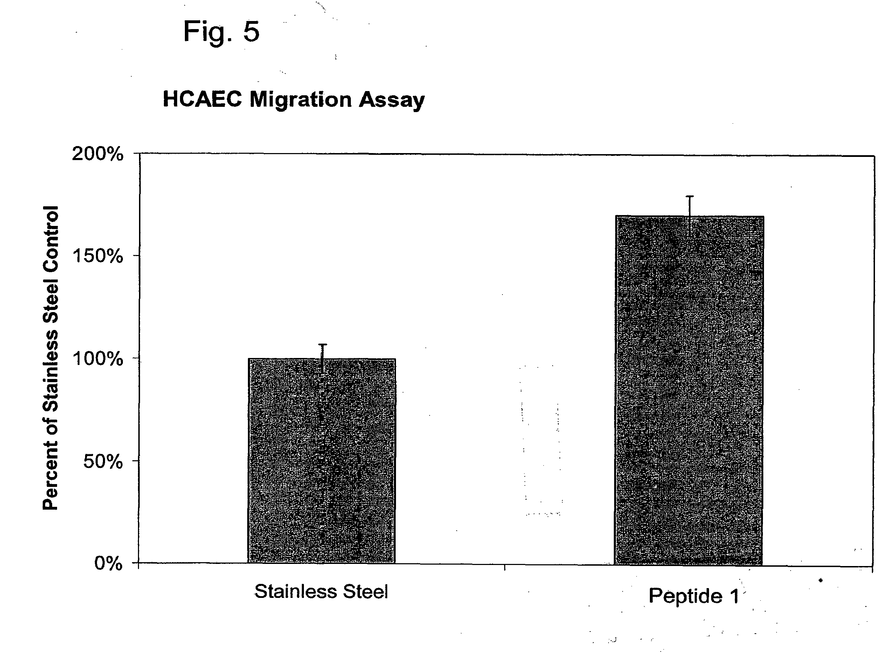

The results are shown in FIG. 5 as the percent of stainless steel control, where uncoated stainless steel was normalized to 1. HCAEC migration was 1.7-fold greater on stainless steel strips pre-coated with PEPTIDE-1 as compared to stainless steel alone. This difference was statistically significant (p<0.01).

### Example 2. Concentration Range Study

A concentration range study was performed to determine an effective concentration for a polypeptide of the disclosure on a stainless steel substrate.

In this example, the surface-binding polypeptide contained DOPA as a surface binding moiety, an (ALA-Aib)3 linker, a peptide fragment (RIGSY), and biotin [(DOPA)2-(ALA-Aib)3-RIGSY-(Biotin)-OH, hereinafter PEPTIDE-2]. PEPTIDE-2 was coated at multiple concentrations between 0.0001 µM and 1000 µM onto 8-mm Liberte WH stents via incubation for 1 hour at room temperature under gentle agitation at 100RPM.

The following procedure was used to for the Biotin Assay: Sequential dilutions of the PEPTIDE-2 in phosphate buffered saline (PBS) was prepared to generate samples having concentrations between 0.0001 µM and 1000 µM. A blank solution with PBS only was also prepared.

8mm Liberte WH stents were placed in the wells of a 48-well polystyrene plate and 240*µ*l of each peptide concentration (including the blank) was then added to each well. The plates were then incubated for at least 1hr at room temperature (RT) with gentle shaking (100 rpm) to allow for binding to occur. Excess peptide solution was removed and discard using a micropipette.

The stents were then washed 3 times with 1.5ml PBS-Tween, with approximately 30sec of shaking at 150 rpm between washes. 1.5ml of 1% bovine serum albumin (BSA) in PBS (blocking solution) was then added to each stent and incubated for 1hr at RT with gentle shaking (100 rpm) to allow for blocking to occur. After 1 hr, excess blocking solution was removed and discarded with a micropipette. The stent was then washed 3 times with 1.5ml PBS-Tween using the wash method described above.

300µl of Streptavidin-AP (alkaline phosphatase) solution was added to each stent at 1/1000 in TBS + 1% BSA and incubated for 30min at room temperature with gentle shaking (100 rpm). Excess Streptavidin solution was then removed and discarded. The stents were then washed 2 times with 1.5ml PBS-Tween and then transferred to a set of new wells on the same plate using tweezers. Once the stents were moved, they were then washed once more with 1.5ml PBS-Tween. 300*µ*l of the chromogen p-nitrophenyl phosphate (pNPP) was added to each stent in the 48 well plate. The plate was then placed on the shaker platform and incubated for 5 minutes at 100 rpm to allow color development to occur. 10*µ*l of sodium hydroxide (NaOH) was then added to the wells of a 96 well tissue culture plate. To stop the color development, 100*µ*l of the solution in each stent containing well was transferred to a well containing NaOH in the 96 well plate.

Optical density (OD) was determined by reading the 96 well plate in a plate reader (Spectramax Plus, Molecular Devices) at 405nm in endpoint mode. The measured OD for the blank was subtracted from the ODs for all other solutions. The resulting ODs were plotted (y-axis) vs. concentration (*µ*M) (log x-axis).

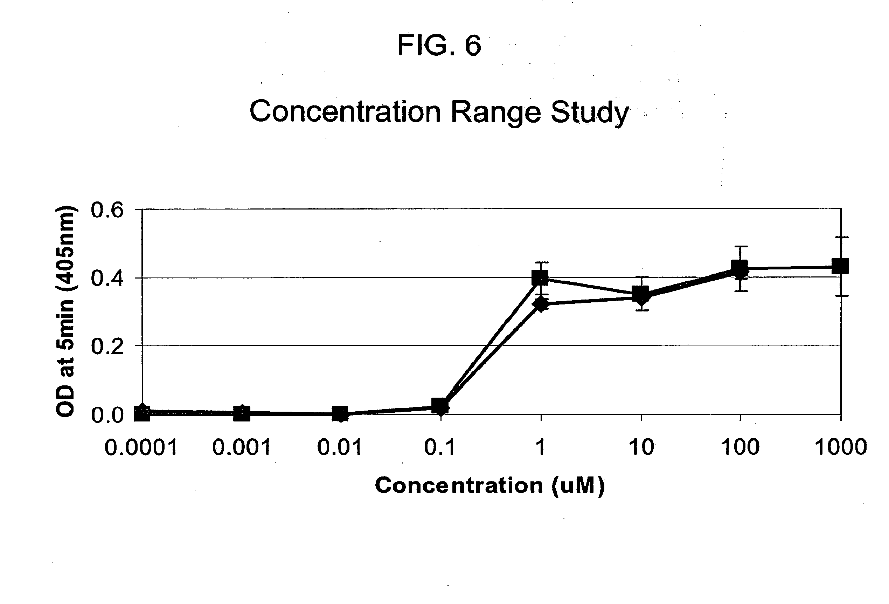

The results are shown in FIG. 6. The two lines represent two separate runs of the same assay. The coating concentration suitable for use in the following experiments was determined to be 10µM. The half-maximal concentration (EC₅₀) was determined to be approximately 0.37µM.

### Example 3. Incubation Time Study

Incubation time studies were performed to investigate peptide affinity for stainless steel over time.

The surface-binding polypeptide of Example 2 (PEPTIDE-2) was applied to a stainless steel substrate (8-mm Liberte WH stent, Boston Scientific) at multiple concentrations between 0.0001 µM and 100µM using the method described in Example 2. The OD at 5 min (405nm) was measured at 1 hr, 4 hr and 24 hrs as described above.

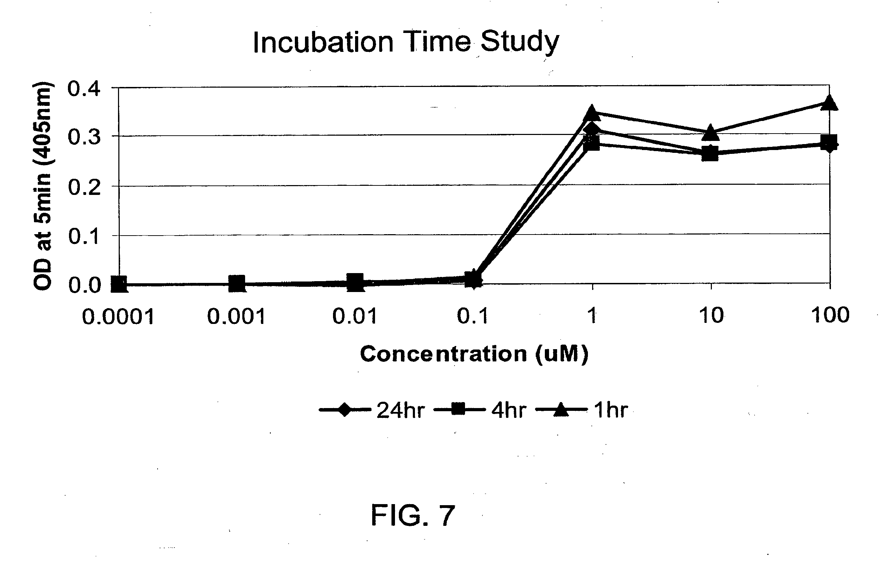

The results are shown in FIG. 7. The EC₅₀ data (24hr=0.27µM; 4hr=0.30µM; 1hr=0.31µM) shows there is only a small change in the affinity of the PEPTIDE-2 to the stainless steel substrate over time.

### Example 4. pH Study

A pH study was conducted to investigate the effects of pH on the affinity of PEPTIDE-2 to a stainless steel substrate (8-mm Liberte WH stent, Boston Scientific).

The PEPTIDE-2 polypeptide of Example 2 was coated on a stainless steel substrate over a concentration range of 0.0001 µM to 100 µM at pH 8, 7, 6, and 4 using the method described in Example 2. The OD at 5 min (405nm) was measured and the EC₅₀ was determined as described above.

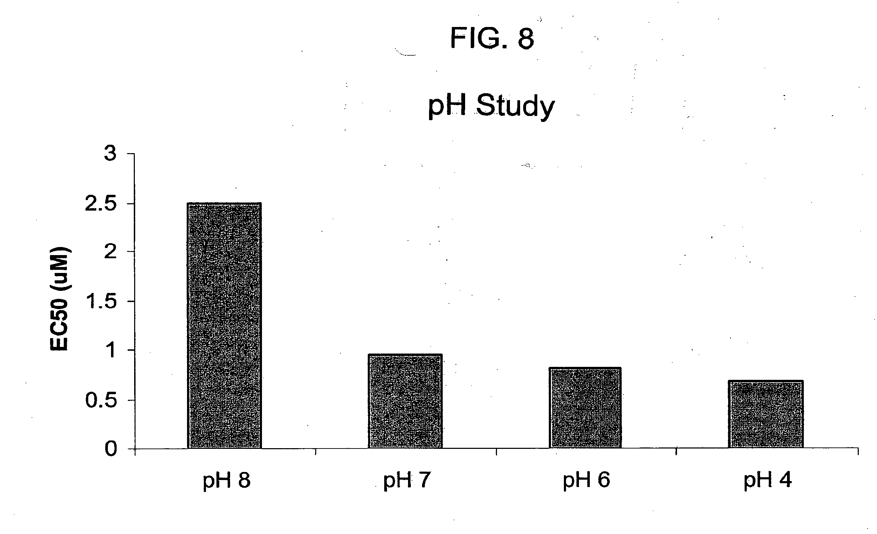

The results are shown in FIG. 8. The EC₅₀s show that while there is a trend, decreasing pH from 7 to 4 does not dramatically alter adhesion.

### Example 5. Sterilization Study

A sterilization study was performed to explore the impact of EtO sterilization on PEPTIDE-2 coated on stainless steel strips. A total of nine (9) stainless steel strips were coated with PEPTIDE-2 at a concentration of 100 µM, as described in Example 2. The nine (9) strips were divided into three categories: (A) three (3) coated strips were stored at 4°C for 2 weeks prior to measuring the OD (referred to as "shelf"); (B) three (3) strips were sterilized using EtO prior to measuring the OD (referred to as "sterilized"); and (C) three (3) strips were "fresh" (i.e., strips that were coated immediately prior to assay and were not allowed to dry in order to evaluate the effects of drying on peptide conformation). The optical density (OD) at 10 min (405 nm) was measured for all nine (9) strips as described above.

The conditions for the sterilization process were as follows:
Process Temperature: 120±5 oF
Initial vacuum pressure: 1.0±0.5 psia
Initial conditioning pressure: 1.25±0.25 psia
Relative humidity (conditioning): 30-100%
Gas inject pressure: 5.4±0.3 psia
Initial gas inject pressure: 1.2+0.3/-0.2 psia
Gas exposure pressure: 14.0 psia
EtO gas concentration: 585 mg/l
Post-vacuum series #1 pressure: 1.0±0.5 psia
Post-vacuum series #2 pressure: 1.0±0.5 psia
Total process time: 22 hours

The affinity of the PEPTIDE-2 peptides was assessed using the Biotin assay described below. It is important to note that the biotin assay indicates the presence of biotin groups but does not survey peptide activity.

Using tweezers an "X" was etched on one side of the stainless steel strip. This side of the strip was assumed to be left uncoated. The strip was placed in a polystyrene Falcon) and 1.5ml of peptide coating solution was added to each vial containing a strip. The vials were then placed in an incubator, oriented so that the etched side of the strip faced upwards. The bottom (unetched) side of the strip in contact with the coating solution was considered the coated side of the strip. The vials were incubated for at least 1hr at room temperature with gentle shaking (100 rpm) to allow for binding to occur. Excess peptide solution was removed and discarded by lifting the strip out of the vial using tweezers and pouring out the solution. After the peptide solution was removed, the stainless steel strip was placed back into the vial. The strips were then washed 3x with 5ml of phosphate buffered saline (PBS)-Tween with 30sec of shaking at 150rpm for each wash. The wash solution was discarded using the pouring method described above.

In the same vial, each strip was blocked with 5mls of BSA blocking solution. The vial was then placed in the incubator with etched side up for a 1hr incubation at room temperature and 100 rpm. After 1 hour, the blocking solution was removed and discarded and the washing procedure described above was repeated.

3.6ml of Streptavidin-AP solution was then added to each vial containing a strip. The vials were incubated with the strip etched side up for 30min at room temperature and 100 rpm. The Streptavidin-AP solution was then removed and discarded. The washing procedure was repeated.

The strips were then loaded into a manifold and 300*µ*l of pNPP was added to each well in the manifold. The manifold was placed on the shaker platform for 10 minutes with gentle agitation (100 rpm) to allow color development to occur.

10*µ*l of NaOH was added to the wells of a 96 well tissue culture plate. To stop the color development, 100*µ*l of the solution in each well was transferred to the corresponding wells containing NaOH in the 96 well plate. The optical density (OD) was determined by reading the 96 well plate containing the solutions in a plate reader at 405nm in endpoint mode.

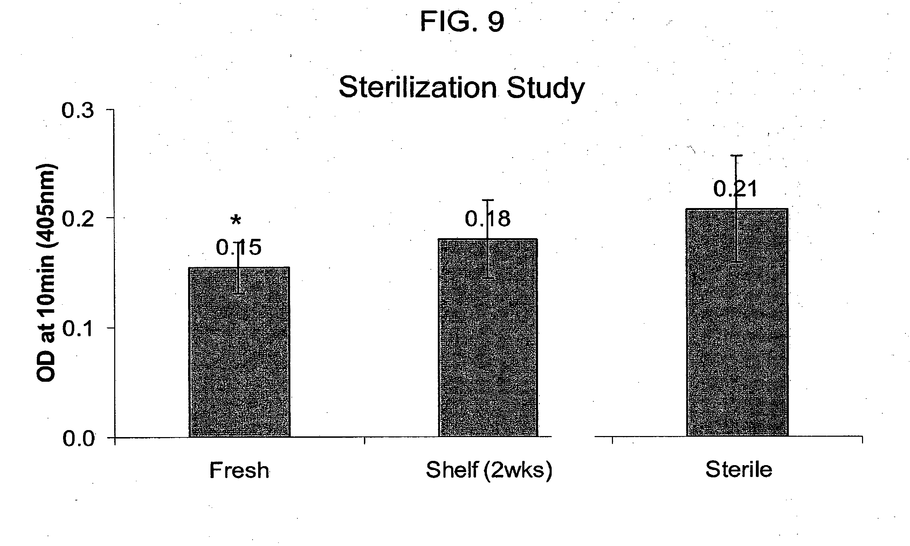

Six (6) measurements were taken for each strip. The results are shown in FIG. 9. The results for the fresh strips are statistically different than the results for the shelf and sterile strips. The shelf and sterile strips are not statistically different from one another. The Biotin assay data indicates that shelving and sterilizing do not negatively impact the amount of peptide present on the stainless steel surface. This assay was not designed to evaluate peptide affinity for the surface.

### Example 6. Fluorescence and Gold Label Imagine

Fluorescence and Gold Label Imaging was used to visualize binding of the surface-binding polypeptides to Liberte WH stents (Boston Scientific, Natick, MA). The Liberte WH stents were coated with 100 µM PEPTIDE-2 using the protocol described in Example 2, above. The coated stents were then labeled using streptavidin conjugated to Alexa-488 (Invitrogen, Carlsbad, CA) or 20 nm immunogold nanoparticles (BBI International, Salida, CO).

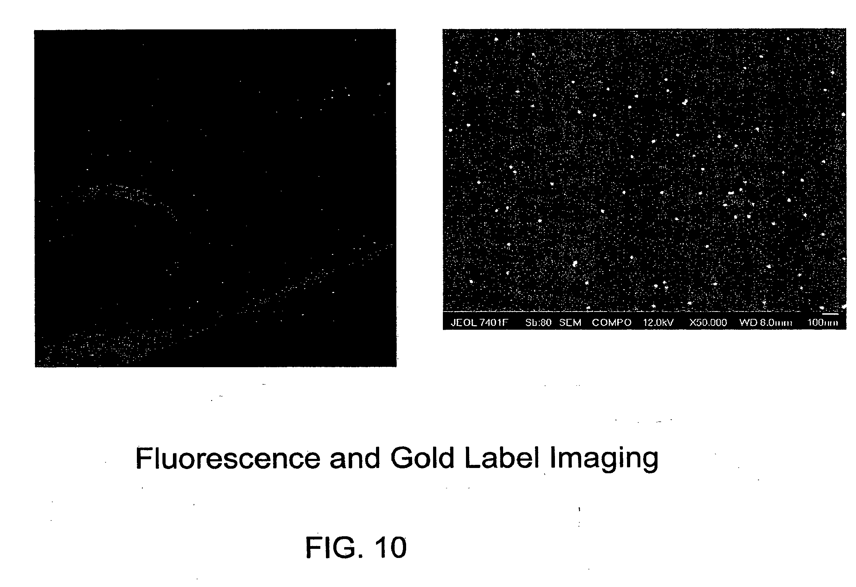

Fluorescence was imaged using an Olympus Fluoview FV1000 confocal microscope. A fluorescent image at 400x is shown in FIG. 10A. The scanning electron microscope (SEM) used to detect the PEPTIDE-2 labeled with the immunogold nanoparticles was a JEOL 7401F. Images were taken at 12.0 kV, WD 8.0 mm. SEM images at 50,000 are shown in FIG. 10B.

### Example 7. Langmuir Isotherm

In this Example, the surface coverage of a polypeptide of the disclosure (PEPTIDE-3) [(Dopa)2-(Ala-Aib)3-RIGSY-OH] was compared to that of a non-specific protein (bovine serum albumin, BSA) using a quartz crystal microbalance with dissipation (QCM-D) technique.

*Reparation of Surfaces:* Stainless steel quartz crystals (Q-Sense, QSX 304) were cleaned with a UV/Ozone treatment (Bioforce, UV Tipcleaner, Model No. UV.TC.110) for 10 minutes. The crystals were then immersed in a 2% sodium dodecyl sulfate (SDS) (MPI, Product No. 811036) in dH₂O solution for 5 minutes, washed thoroughly with dH₂O, N₂ dried, and UV/Ozone treated for an additional 10 minutes.

*Surface Characterization:* The QCM-D measurements (Q-Sense E4) were made with a solution flow rate of 100*µ*L per minute. The experiment was temperature controlled at 25°C.

In the experiments, four samples were tested at one time using the QCM-D. Each experiment included running PBS for approximately 10 minutes to establish a stable baseline. The peptide solutions were then added at various concentrations until the change in frequency was 2Hz per 30 minutes or less. The final step was to wash with PBS for 30 minutes or until the change in frequency was 2Hz per 30 minutes or less. For analysis purposes (Sauerbrey model, Q-Tools), generally the third overtone was used from each sample.

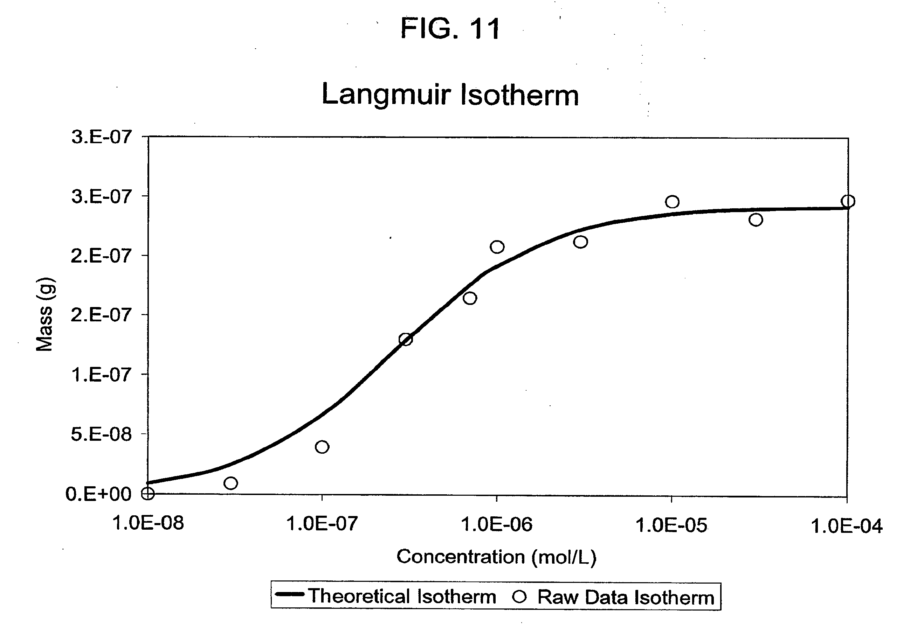

The K_{d} and EC₅₀ were determined and the results are shown in FIG. 11. A K_{d} value of 0.26µM was found or an EC₅₀ of 0.28µM. The theoretical fit indicates that the adsorption mechanism follows a Langmuir Isotherm suggesting that a monolayer is formed. In comparison to the non-specific protein, BSA, which was determined to have an EC₅₀ of no less than 100µM, PEPTIDE-2 shows approx. 3 orders of magnitude greater affinity for stainless steel.

## Claims

1. A stent having a metallic surface and a coating on the metallic surface, the coating comprising:
surface-binding cell adhesion polypeptides disposed on at least a portion of the metallic surface, wherein the surface-binding cell adhesion polypeptides comprise:
a surface-binding moiety comprising a catechol moiety bound to the metallic surface; and
a cell adhesion polypeptide attached to the surface-binding moiety,
wherein the cell adhesion polypeptide comprises an extracellular matrix protein or a binding domain fragment thereof.

2. The stent of claim 1, wherein the metallic surface is a luminal surface.

3. The stent of claim 1, wherein the cell adhesion polypeptide is attached to the surface-binding moiety via a linker.

4. The stent of claim 1, wherein the catechol moiety is selected from the group consisting of a DOPA moiety, a moiety of formula I, a moiety of formula II, and a moiety of formula III: 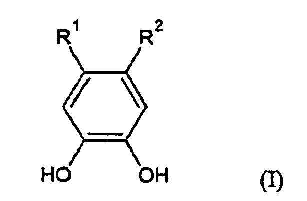 wherein R¹ and R² are individually selected from: the cell adhesion polypeptide, a linker, an additional surface-binding moiety, H, or combinations thereof; wherein at least one of R¹ and R² is directly or indirectly attached to the cell adhesion polypeptide; 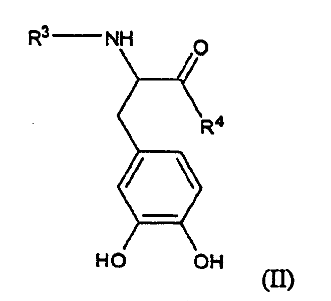 wherein R³ and R⁴ are individually selected from: the cell adhesion polypeptide, a linker, an additional surface-binding moiety, H, or combinations thereof; wherein at least one of R³ and R⁴ is directly or indirectly attached to the cell adhesion polypeptide; or 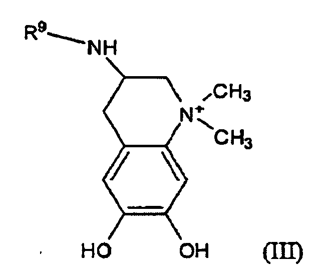 wherein R⁹ is the cell adhesion polypeptide, a linker, an additional surface-binding moiety, H, or combinations thereof; wherein R⁹ is directly or indirectly attached to the cell adhesion polypeptide.

5. The stent of claim 4, wherein the catechol moiety is (DOPA-Lys)₂.

6. A method for coating a stent having a metallic surface comprising:
contacting a surface of the stent with surface-binding cell adhesion polypeptides, wherein the surface-binding cell adhesion polypeptides comprise at least one cell adhesion polypeptide and at least one surface-binding moiety comprising a catechol moiety, wherein the cell adhesion polypeptide comprises an extracellular matrix protein or a binding domain fragment thereof; and
binding the surface-binding cell adhesion polypeptides to at least a portion of the metallic surface.

7. The method of claim 6, wherein contacting the stent with surface-binding cell adhesion polypeptides comprises dipping the stent into a solution including surface-binding cell adhesion polypeptides.

## Patentansprüche

1. Ein Stent mit einer metallischen Oberfläche und einer Beschichtung auf der metallischen Oberfläche, wobei die Beschichtung umfasst:
oberflächenbindende Zelladhäsionspolypeptide, welche auf mindestens einem Teil der metallischen Oberfläche angeordnet sind, wobei die oberflächenbindenden Zelladhäsionspolypeptide umfassen:
eine oberflächenbindende Einheit, umfassend eine Catecholeinheit, welche an die metallische Oberfläche gebunden ist; und
ein Zelladhäsionspolypeptid, welches an die oberflächenbindende Einheit gebunden ist, wobei das Zelladhäsionspolypeptid ein extrazelluläres Matrixprotein oder ein Bindungsdomänenfragment davon umfasst.

2. Der Stent nach Anspruch 1, wobei die metallische Oberfläche eine luminale Oberfläche ist.

3. Der Stent nach Anspruch 1, wobei das Zelladhäsionspolypeptid über einen Linker an die oberflächenbindende Einheit gebunden ist.

4. Der Stent nach Anspruch 1, wobei die Catecholeinheit aus der Gruppe, bestehend aus einer DOPA-Einheit, einer Einheit der Formel I, einer Einheit der Formel II und einer Einheit der Formel III, ausgewählt ist: 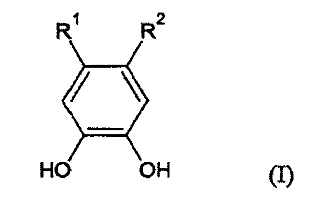 wobei R¹ und R² einzeln ausgewählt sind aus: dem Zelladhäsionspolypeptid, einem Linker, einer zusätzlichen oberflächenbindenden Einheit, H oder Kombinationen davon; wobei mindestens eines von R¹ und R² direkt oder indirekt an das Zelladhäsionspolypeptid gebunden ist; 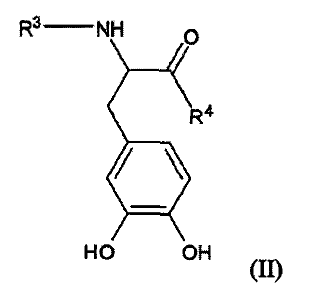 wobei R³ und R⁴ einzeln ausgewählt sind aus: dem Zelladhäsionspolypeptid, einem Linker, einer zusätzlichen oberflächenbindenden Einheit, H oder Kombinationen davon; wobei mindestens eines von R³ und R⁴ direkt oder indirekt an das Zelladhäsionspolypeptid gebunden ist; oder 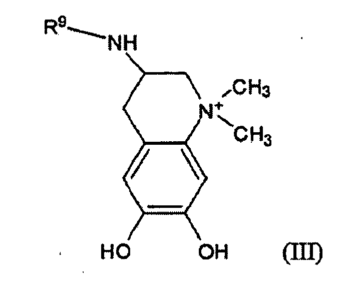 wobei R⁹ das Zelladhäsionspolypeptid, ein Linker, eine zusätzliche oberflächenbindende Einheit, H oder Kombinationen davon ist; wobei R⁹ direkt oder indirekt an das Zelladhäsionspolypeptid gebunden ist.

5. Der Stent nach Anspruch 4, wobei die Catecholeinheit (DOPA-Lys)₂ ist.

6. Ein Verfahren zur Beschichtung eines Stents, der eine metallische Oberfläche aufweist, umfassend:
In Kontakt bringen einer Oberfläche des Stents mit oberflächenbindenden Zelladhäsionspolypeptiden, wobei die oberflächenbindenden Zelladhäsionspolypeptide mindestens ein Zelladhäsionspolypeptid und mindestens eine oberflächenbindende Einheit, umfassend eine Catecholeinheit, umfassen, wobei das Zelladhäsionspolypeptid ein extrazelluläres Matrixprotein oder ein Bindungsdomänenfragment davon umfasst; und
Binden der oberflächenbindenden Zelladhäsionspolypeptide an mindestens einen Teil der metallischen Oberfläche.

7. Das Verfahren nach Anspruch 6, wobei in Kontakt bringen des Stents mit oberflächenbindenden Zelladhäsionspolypeptiden Eintauchen des Stents in eine oberflächenbindende Zelladhäsionspolypeptide enthaltende Lösung umfasst.

## Revendications

1. Stent comportant une surface métallique et un revêtement sur la surface métallique, le revêtement comprenant :
des polypeptides d'adhésion cellulaire se liant à une surface disposés sur au moins une partie de la surface métallique, où les polypeptides d'adhésion cellulaire se liant à une surface comprennent :
un fragment se liant à une surface comprenant un fragment catéchol lié à la surface métallique ; et
un polypeptide d'adhésion cellulaire attaché au fragment se liant à une surface, où le polypeptide d'adhésion cellulaire comprend une protéine de la matrice extracellulaire ou un fragment du domaine de liaison de celle-ci.

2. Stent selon la revendication 1, où la surface métallique est une surface luminale.

3. Stent selon la revendication 1, où le polypeptide d'adhésion cellulaire est attaché au fragment se liant à une surface via un segment de liaison.

4. Stent selon la revendication 1, où le fragment catéchol est sélectionné dans le groupe consistant en un fragment DOPA, un fragment de formule I, un fragment de formule II, et un fragment de formule III : 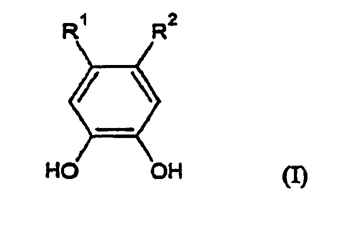 où R¹ et R² sont individuellement sélectionnés parmi : le polypeptide d'adhésion cellulaire, un segment de liaison, un fragment se liant à une surface supplémentaire, H, ou des combinaisons de ceux-ci ; où au moins l'un de R¹ et R² est directement ou indirectement attaché au polypeptide d'adhésion cellulaire ; 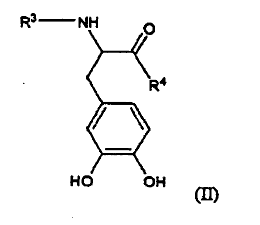 où R³ et R⁴ sont individuellement sélectionnés parmi : le polypeptide d'adhésion cellulaire, un segment de liaison, un fragment se liant à une surface supplémentaire, H, ou des combinaisons de ceux-ci ; où au moins l'un de R³ et R⁴ est directement ou indirectement attaché au polypeptide d'adhésion cellulaire ; ou 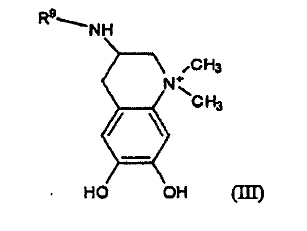 où R⁹ est le polypeptide d'adhésion cellulaire, un segment de liaison, un fragment se liant à une surface supplémentaire, H, ou des combinaisons de ceux-ci ; où R⁹ est directement ou indirectement attaché au polypeptide d'adhésion cellulaire.

5. Stent selon la revendication 4, où le fragment catéchol est (DOPA-Lys)₂.

6. Procédé pour appliquer un revêtement sur un stent comportant une surface métallique comprenant :
la mise en contact d'une surface du stent avec des polypeptides d'adhésion cellulaire se liant à une surface, où les polypeptides d'adhésion cellulaire se liant à une surface comprennent au moins un polypeptide d'adhésion cellulaire et au moins un fragment se liant à une surface comprenant un fragment catéchol, où le polypeptide d'adhésion cellulaire comprend une protéine de la matrice extracellulaire ou un fragment du domaine de liaison de celle-ci ; et
la liaison des polypeptides d'adhésion cellulaire se liant à une surface à au moins une partie de la surface métallique.

7. Procédé selon la revendication 6, dans lequel la mise en contact du stent avec des polypeptides d'adhésion cellulaire se liant à une surface comprend l'immersion du stent dans une solution comprenant des polypeptides d'adhésion cellulaire se liant à une surface.
